(19) **Europäisches Patentamt**
**European Patent Office**
**Office européen des brevets**

(11) **EP 1 416 846 B1**

(12) **EUROPÄISCHE PATENTSCHRIFT**

(45) Veröffentlichungstag und Bekanntmachung des Hinweises auf die Patenterteilung:
**31.10.2007 Patentblatt 2007/44**

(21) Anmeldenummer: **02794760.5**

(22) Anmeldetag: **08.08.2002**

(51) Int Cl.:
***A61B 5/00*** *(2006.01)*

(86) Internationale Anmeldenummer:
**PCT/EP2002/008855**

(87) Internationale Veröffentlichungsnummer:
**WO 2003/015626 (27.02.2003 Gazette 2003/09)**

(54) **VERFAHREN UND VORRICHTUNG ZUM BESTIMMEN EINES KENNWERTES FÜR DIE DURCHBLUTUNG VON VERÄNDERTEN GEWEBEBEREICHEN IN ORGANEN VON LEBEWESEN**

METHOD AND DEVICE FOR DETERMINING A CHARACTERISTIC VALUE RELATING TO THE BLOOD CIRCULATION OF MODIFIED TISSUE REGIONS IN ORGANS OF LIVING BEINGS

PROCEDE ET DISPOSITIF PERMETTANT DE DETERMINER UNE VALEUR CARACTERISTIQUE RELATIVE A LA CIRCULATION SANGUINE DE REGIONS TISSULAIRES MODIFIEES DANS DES ORGANES D'ETRES VIVANTS

(84) Benannte Vertragsstaaten:
**AT BE BG CH CY CZ DE DK EE ES FI FR GB GR IE IT LI LU MC NL PT SE SK TR**

(30) Priorität: **14.08.2001 DE 10140924**

(43) Veröffentlichungstag der Anmeldung:
**12.05.2004 Patentblatt 2004/20**

(73) Patentinhaber: **Eberhard-Karls-Universität Tübingen**
**Universitätsklinikum**
**72076 Tübingen (DE)**

(72) Erfinder:
 • **BRÄUER, Kurt**
  **72076 Tübingen (DE)**
 • **HÄFNER, Hans-Martin**
  **70619 Stuttgart (DE)**

(74) Vertreter: **Otten, Hajo et al**
**Witte, Weller & Partner**
**Patentanwälte,**
**Postfach 105462**
**70047 Stuttgart (DE)**

(56) Entgegenhaltungen:
 • TUR E ET AL: "CUTANEOUS BLOOD FLOW MEASUREMENTS FOR THE DETECTION OF MALIGNANCY IN PIGMENTED SKIN LESIONS" DERMATOLOGY (BASEL), Bd. 184, Nr. 1, 1992, Seiten 8-11, XP008014346 ISSN: 1018-8665
 • KVERNMO H D ET AL: "OSCILLATIONS IN THE HUMAN CUTANEOUS BLOOD PERFUSION SIGNAL MODIFIED BY ENDOTHELIUM-DEPENDENT AND ENDOTHELIUM-INDEPENDENT VASODILATORS" MICROVASCULAR RESEARCH, ACADEMIC PRESS, US, Bd. 57, 1999, Seiten 298-309, XP001090835 ISSN: 0026-2862 in der Anmeldung erwähnt
 • STUECKER M ET AL: "Blood flow compared in benign melanocytic naevi, malignant melanomas and basal cell carcinomas." CLINICAL AND EXPERIMENTAL DERMATOLOGY, Bd. 24, Nr. 2, März 1999 (1999-03), Seiten 107-111, XP002232886 ISSN: 0307-6938

**Beschreibung**

Die Erfindung betrifft ein Verfahren zum Bestimmen eines Kennwertes für die Durchblutung von veränderten Gewebebereichen in Organen von Lebewesen.

Die Erfindung betrifft weiterhin eine Vorrichtung zum Bestimmen eines Kennwertes für die Durchblutung von veränderten Gewebebereichen in Organen von Lebewesen.

Es ist in der Dermatologie bekannt, zum Untersuchen von pigmentierten Gewebebereichen verschiedene Untersuchungsmethoden einzusetzen, z.B. die Auflicht-Oberflächenmikroskopie, die Epilumineszenz-Mikroskopie, die Dermatoskopie usw. Diesen Untersuchungsmethoden liegt die Erkenntnis zugrunde, daß die Morphologie unterschiedlicher Gewebebereiche, speziell die Mikrozirkulation innerhalb pigmentierter Gewebebereiche durch Kennwerte charakterisiert werden kann, die als Beurteilungskriterien für diesen Gewebebereich herangezogen werden können. In diesem Zusammenhang ist bekannt, daß die Kapillargefäße bei malignen Tumoren krankhaft verändert sind und sich eine sogenannte Hypervaskularisation im Randbereich des tumorösen Gewebes herausbildet, wobei kapillare Blutgefäße in Richtung auf den Tumor wachsen. Dieses Phänomen wird als Neoangiogenese ("einsprossende Gefäße") bezeichnet.

Es ist bekannt, zur Untersuchung der Perfusion in menschlichem Hautgewebe Laserdoppler-Flowmeter einzusetzen (Kvernmo H.D. et al., Microvascular Research 57, Seite 298-309, 1999). In diesem Zusammenhang ist es auch bekannt, die Laserdoppler-Perfusionssignale, die als Volumenstrom über der Zeit erfaßt werden, aufzuarbeiten, und zwar mittels einer sogenannten Wavelet-Analyse. Unter einer Wavelet-Analyse versteht man ein Verfahren, bei dem ein zeitvariantes Signal dreidimensional dargestellt wird, und zwar über den Achsen Frequenz oder Skalierung, Zeit und Signalamplitude. Die Wavelet-Analyse wird bevorzugt bei solchen zeitvarianten Signalen verwendet, bei denen sich mehrere periodische oder quasi-periodische Vorgänge überlagern, möglicherweise zusammen mit weiteren stochastischen Prozessen. Die Wavelet-Analyse gestattet es in einem solchen Fall, bestimmte Frequenz- oder Skalierungsbereiche, in denen einzelne periodische oder quasi-periodische Signale auftreten, gezielt herauszufiltern oder gezielt zu unterdrücken.

Die dreidimensionale Darstellung eines Laserdoppler-Perfusionssignals mittels Wavelet-Analyse wird nachstehend als "Vasomotionsfeld" bezeichnet.

In dem weiter oben erwähnten Aufsatz von Kvernmo et al. wird die Perfusion in menschlichem Hautgewebe in Abhängigkeit von der Gabe bestimmter gefäßerweiternder Arzneimittel untersucht. Die im Rahmen dieser Arbeit bestimmten Kennwerte beziehen sich daher ausschließlich auf die Auswirkung dieser Medikamentengabe. Kennwerte, die das Durchblutungsverhalten des Gewebes selbst, insbesondere im Vergleich zwischen pigmentierten und nicht-pigmentierten Gewebebereichen betreffen, sind in dieser Arbeit nicht offenbart.

Auch aus TUR E ET AL: 'CUTANEOUS BLOOD FLOW MEASUREMENTS FOR THE DETECTION OF MALIGNANCY IN PIGMENTED SKIN LESIONS' DERMATOLOGY (BASEL), Bd. 184, Nr. 1, 1992, Seiten 8-11, ist ein Verfahren zur Bestimmung eines Kennwertes ("probability of malignancy") für die Durchblutung von veränderten Hautbereichen bekannt.

Der Erfindung liegt die Aufgabe zugrunde, ein Verfahren und eine Vorrichtung der eingangs genannten Art dahingehend weiterzubilden, daß Kennwerte bestimmt werden können, die gerade für die Durchblutung von veränderten, insbesondere von pigmentierten Gewebebereichen in inneren Organen, insbesondere der Haut charakteristisch sind.

Ein Verfahren der eingangs genannten Art umfasst die folgenden Schritte:

a) Bestimmen eines ersten Volumenstroms des Blutes mittels Laser-Fluxmetrie in einem ersten Meßpunkt innerhalb des veränderten Gewebebereichs als erster Meßverlauf eines Volumenstrom-Signals in Abhängigkeit von der Zeit;

b) Bestimmen eines zweiten Volumenstroms des Blutes mittels Laser-Fluxmetrie in einem zweiten Meßpunkt innerhalb eines nicht-veränderten Gewebebereichs als zweiter Meßverlauf eines Volumenstrom-Signals in Abhängigkeit von der Zeit;

c) Ausführen jeweils einer Wavelet-Analyse der Meßverläufe als dreidimensionale Darstellung der Signalintensität über einer Frequenz- oder Skalierungsachse und der Zeit;

d) Bestimmen jeweils des Verlaufs einer Vasomotionsenergie über der Frequenz- oder Skalierungsachse für die der Wavelet-Analyse unterworfenen Meßverläufe, wobei die Vasomotionsenergie das Integral der Signalintensität über der Zeit für einen bestimmten Frequenz- oder Skalierungswert ist;

e) Subtrahieren des Verlaufs der Vasomotionsenergie des zweiten Meßverlaufs vom Verlauf der Vasomotionsenergie des ersten Meßverlaufs, derart, daß ein erster Differenzverlauf gebildet wird;

f) Aufintegrieren des Differenzverlaufs zum Erhalten des Kennwertes.

Bei einer bevorzugten Variante des eingangs genannten Verfahrens wird die der Erfindung zugrunde liegende Aufgabe durch die folgenden Merkmale gelöst:

a) Bestimmen eines ersten Volumenstroms des Blutes mittels Laser-Fluxmetrie in einem ersten Meßpunkt innerhalb des veränderten Gewebebereichs als erster Meßverlauf eines Volumenstrom-Signals in Abhängigkeit von der Zeit;

b) Bestimmen eines zweiten Volumenstroms des Blutes mittels Laser-Fluxmetrie in einem zweiten Meßpunkt innerhalb eines nicht-veränderten Gewebebereichs als zweiter Meßverlauf eines Volumenstrom-Signals in Abhängigkeit von der Zeit;

c) Bestimmen eines dritten Volumenstroms des Blutes mittels Laser-Fluxmetrie in einem dritten Meßpunkt im Übergang zwischen dem veränderten Gewebebereich und dem umgebenden, nicht-veränderten Gewebebereich als dritter Meßverlauf eines Volumenstrom-Signals in Abhängigkeit von der Zeit;

d) Ausführen jeweils einer Wavelet-Analyse der Meßverläufe als dreidimensionale Darstellung der Signalintensität über einer Frequenz- oder Skalierungsachse und der Zeit;

e) Bestimmen jeweils des Verlaufs einer Vasomotionsenergie über der Frequenz- oder Skalierungsachse für die der Wavelet-Analyse unterworfenen Meßverläufe, wobei die Vasomotionsenergie das Integral der Signalintensität über der Zeit für einen bestimmten Frequenz- oder Skalierungswert ist;

f) Subtrahieren des Verlaufs der Vasomotionsenergie des dritten Meßverlaufs vom Verlauf der Vasomotionsenergie des zweiten Meßverlaufs, derart, daß ein erster Differenzverlauf gebildet wird;

g) Subtrahieren des Verlaufs der Vasomotionsenergie des ersten Meßverlaufs vom Verlauf der Vasomotionsenergie des zweiten Meßverlaufs, derart, daß ein zweiter Differenzverlauf gebildet wird;

h) Bilden eines Mittelwertes des ersten und des zweiten Differenzverlaufs;

i) Aufintegrieren des Mittelwertes zum Erhalten des Kennwertes.

Eine Vorrichtung der eingangs genannten Art umfasst die folgenden Merkmale:

Vorrichtung zum Bestimmen eines Kennwertes für die Durchblutung von veränderten Gewebebereichen in Organen von Lebewesen mit:

a) einem Laser-Fluxmeter zum Bestimmen

- eines ersten Volumenstroms des Blutes in einem ersten Meßpunkt innerhalb des veränderten Gewebebereichs als erster Meßverlauf eines Volumenstrom-Signals in Abhängigkeit von der Zeit;

- eines zweiten Volumenstroms des Blutes in einem zweiten Meßpunkt innerhalb eines nicht-veränderten Gewebebereichs als zweiter Meßverlauf eines Volumenstrom-Signals in Abhängigkeit von der Zeit;

b) Mitteln zum Ausführen jeweils einer Wavelet-Analyse der Meßverläufe als dreidimensionale Darstellung der Signalintensität über einer Skalierungsachse und der Zeit;

c) Mitteln zum Bestimmen jeweils des Verlaufs einer Vasomotionsenergie über der Frequenz- oder Skalierungsachse für die der Wavelet-Analyse unterworfenen Meßverläufe, wobei die Vasomotionsenergie das Integral der Signalintensität über der Zeit für einen bestimmten Frequenz- oder Skalierungswert ist;

d) Mitteln zum Subtrahieren des Verlaufs der Vasomotionsenergie des ersten Meßverlaufs vom Verlauf der Vasomotionsenergie des zweiten Meßverlaufs, derart, daß ein Differenzverlauf gebildet wird;

e) Mitteln zum Aufintegrieren des Differenzverlaufs zum Erhalten des Kennwertes.

Bei einer bevorzugten Variante der eingangs genannten Vorrichtung wird die der Erfindung zugrunde liegende Aufgabe durch die folgenden Merkmale gelöst:

Vorrichtung zum Bestimmen eines Kennwertes für die Durchblutung von veränderten Gewebebereichen in Organen von Lebewesen mit:

a) einem Laser-Fluxmeter zum Bestimmen

- eines ersten Volumenstroms des Blutes in einem ersten Meßpunkt innerhalb des veränderten Gewebebereichs als erster Meßverlauf eines Volumenstrom-Signals in Abhängigkeit von der Zeit;

- eines zweiten Volumenstroms des Blutes in einem zweiten Meßpunkt innerhalb eines nicht-veränderten Gewebebereichs als zweiter Meßverlauf eines Volumenstrom-Signals in Abhängigkeit von der Zeit;

- eines dritten Volumenstroms des Blutes in einem dritten Meßpunkt im Übergang zwischen dem veränderten Gewebebereich und dem umgebenden, nicht-veränderten Gewebebereich als dritter Meßverlauf eines Volumenstrom-Signals in Abhängigkeit von der Zeit;

b) Mitteln zum Ausführen jeweils einer Wavelet-Analyse der Meßverläufe als dreidimensionale Darstellung der Signalintensität über einer Frequenz- oder Skalierungsachse und der Zeit;

c) Mitteln zum Bestimmen jeweils des Verlaufs einer Vasomotionsenergie über der Frequenz- oder Skalierungsachse für die der Wavelet-Analyse unterworfenen Meßverläufe, wobei die Vasomotionsenergie das Integral der Signalintensität über der Zeit für einen bestimmten Frequenz- oder Skalierungswert ist;

d) Mitteln zum Subtrahieren des Verlaufs der Vasomotionsenergie des dritten Meßverlaufs vom Verlauf der Vasomotionsenergie des zweiten Meßverlaufs, derart, daß ein erster Differenzverlauf gebildet wird;

e) Mitteln zum Subtrahieren des Verlaufs der Vasomotionsenergie des ersten Meßverlaufs vom Verlauf der Vasomotionsenergie des zweiten Meßverlaufs, derart, daß ein zweiter Differenzverlauf gebildet wird;

f) Mitteln zum Bilden eines Mittelwertes des ersten und des zweiten Differenzverlaufs;

g) Mitteln zum Aufintegrieren des Mittelwertes zum Erhalten des Kennwertes.

Die der Erfindung zugrunde liegende Aufgabe wird auf diese Weise vollkommen gelöst.

Es hat sich nämlich gezeigt, daß mit den vorstehend aufgelisteten Merkmalen Kennwerte bestimmt werden können, die eine praktisch 100%ige Bestimmung der Eigenschaften des untersuchten veränderten Gewebereichs zulassen. Diese Zuverlässigkeit wurde auch in einer klinischen Studie bestätigt.

Bei einer bevorzugten Weiterbildung des erfindungsgemäßen Verfahrens kann der Kennwert mit einem Referenzwert verglichen werden.

Diese Maßnahme hat den Vorteil, daß eine Zuordnung des ermittelten Kennwertes zu klinisch ermittelten Grenzwerten möglich ist, um daraus Schlußfolgerungen für die Beschreibung des Zustandes des veränderten Gewebebereiches zu ziehen.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:

Fig. 1          eine äußerst schematisierte Darstellung eines Hautbereichs, in dem sich ein pigmentierter Gewebebereich befindet, unter Angabe von erfindungsgemäß gewählten Meßpunkten;

Fig. 2A und 2B    Laserdoppler-Perfusionssignale, wie sie an den Meßpunkten gemäß Fig. 1 als Volumenstrom des Blutes über der Zeit bestimmt werden können;

Fig. 3A bis 3D    vier Vasomotionsfelder, wie sie sich durch Wavelet-Analyse aus Laserdoppler-Perfusionssignalen ergeben;

Fig. 4         das Vasomotionsfeld gemäß Fig. 3D, in größerem Maßstab und zur Erläuterung weiterer Einzelheiten;

Fig. 5         den graphischen Verlauf einer Vasomotionsenergie über einer Skalierungsachse für einen malignen pigmentierten Gewebebereich, im Vergleich zu umgebendem Gewebe;

Fig. 6         eine Darstellung, ähnlich Fig. 5, jedoch für einen benignen pigmentierten Gewebebereich;

Fig. 7         ein Balkendiagramm zur Erläuterung einer erfindungsgemäß durchgeführten klinischen Studie.

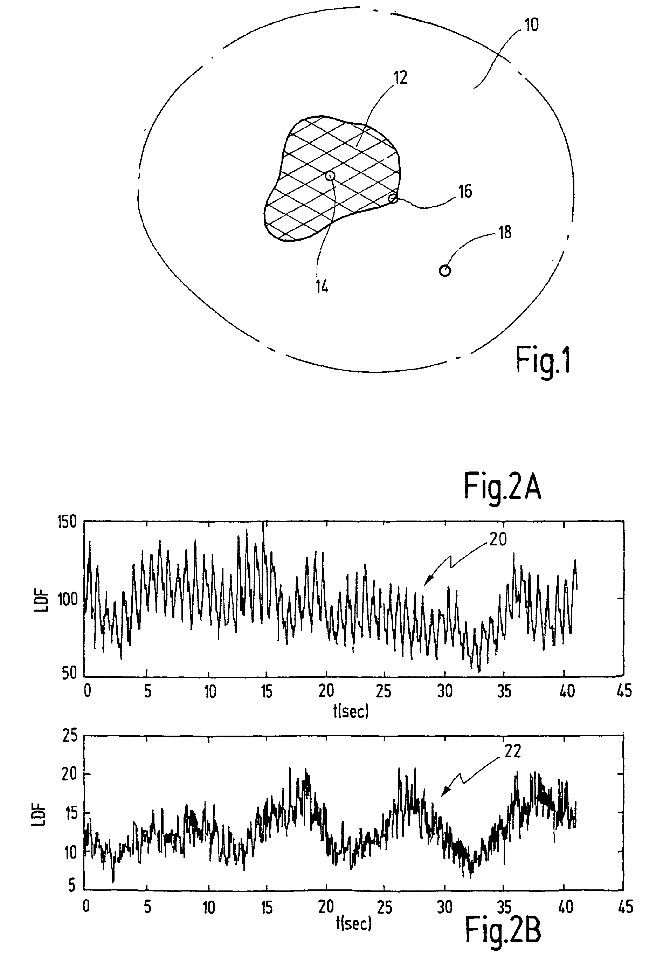

In Fig. 1 bezeichnet 10 äußerst schematisiert einen Hautbereich eines Menschen. Innerhalb des Hautbereiches 10 befindet sich ein veränderter, im dargestellten Beispielsfall ein pigmentierter Gewebebereich 12. Der pigmentierte Gewebebereich 12 kann benigne oder maligne sein. Zu den benignen Erscheinungsformen gehören Sommersprossen, Leberflecken und dergleichen (melanozytische Naevi), während zu den malignen Erscheinungsformen die verschiedenen Arten des Hautkrebses (Melanome) gehören. Der Hautbereich 10 steht beispielhaft für ein beliebiges Organ, insbesondere inneres Organ, eines Lebewesens.

In Fig. 1 ist mit 14 ein erster Meßpunkt im Zentrum des pigmentierten Gewebebereichs 12, mit 16 ein zweiter Meßpunkt am Rande des pigmentierten Gewebebereichs 12 sowie mit 18 ein dritter Meßpunkt im umgebenden, nicht-pigmentierten Gewebebereich bezeichnet. Der zweite Meßpunkt 16 ist für die Durchführung des Verfahrens nicht zwingend erforderlich, wird aber bei bevorzugten Varianten des Verfahrens ausgewertet.

An den zwei Meßpunkten 14, 18 bzw. den drei Meßpunkten 14, 16 und 18 werden nun mittels eines Laserdoppler-Fluxmeters Messungen durchgeführt. Derartige Laserdoppler-Fluxmeter sind dem Fachmann bekannt und werden z.B. unter der Gerätebezeichnung "Periflux System 5000" von der Firma Perimed, Stockholm, Schweden vertrieben.

Bei Laserdoppler-Fluxmetern wird ein Laserstrahl in die zu untersuchenden Gefäße geleitet. Infolge des Dopplereffektes ist das von den bewegten Blutkörperchen reflektierte Licht in seiner Frequenz, d.h. in Farbe aber auch in Intensität verschoben. Auf diese Weise können Messungen des Volumenstroms des Blutes durchgeführt werden. Es ergeben sich dann Meßverläufe eines Volumenstrom-Signals in Abhängigkeit von der Zeit.

In den Fig. 2A und 2B sind zwei derartige Meßverläufe 20 und 22 für die Meßpunkte 14 und 18 aus Fig. 1 dargestellt. Man erkennt deutlich, daß im ersten Meßpunkt 14 eine größere und im dritten Meßpunkt 18 eine kleinere Signalamplitude erhalten wird. Ferner läßt sich den Meßverläufen 20 und 22 entnehmen, daß offensichtlich mehrere periodische und quasi-periodische Vorgänge einem stochastischen Vorgang überlagert sind. Die periodischen bzw. quasi-periodischen Vorgänge sind dabei die physiologischen Grundfunktionen des menschlichen Körpers (Atmung, Pulsschlag usw.). Diese Vorgänge ändern sich bekanntlich mit der Zeit, so daß die Meßverläufe gemäß den Fig. 2A und 2B an sich wenig aussagekräftig sind. Es läßt sich lediglich daraus schließen, daß der pigmentierte Gewebebereich 12 offensichtlich wesentlich stärker durchblutet ist als der umgebende nicht-pigmentierte Gewebebereich.

Um die Aussagekraft der erfaßten Meßverläufe 20 und 22 zu verbessern, wird erfindungsgemäß eine spezielle Wavelet-Analyse durchgeführt. Wie bereits weiter oben erwähnt wurde, ist die Wavelet-Analyse ein an sich bekanntes Werkzeug in der Signalverarbeitung, um bei Meßverläufen der hier vorliegenden Art einen Zeitverlauf in eine dreidimensionale Darstellung mit den Achsen Zeit, Frequenz oder Skalierung und Signalintensität zu überführen. Aus der Darstellung der Wavelet-Analyse lassen sich dann die verschiedenen periodischen, quasi-periodischen und stochastischen Prozesse besser erkennen und je nach Bedarf herausfiltern bzw. unterdrücken.

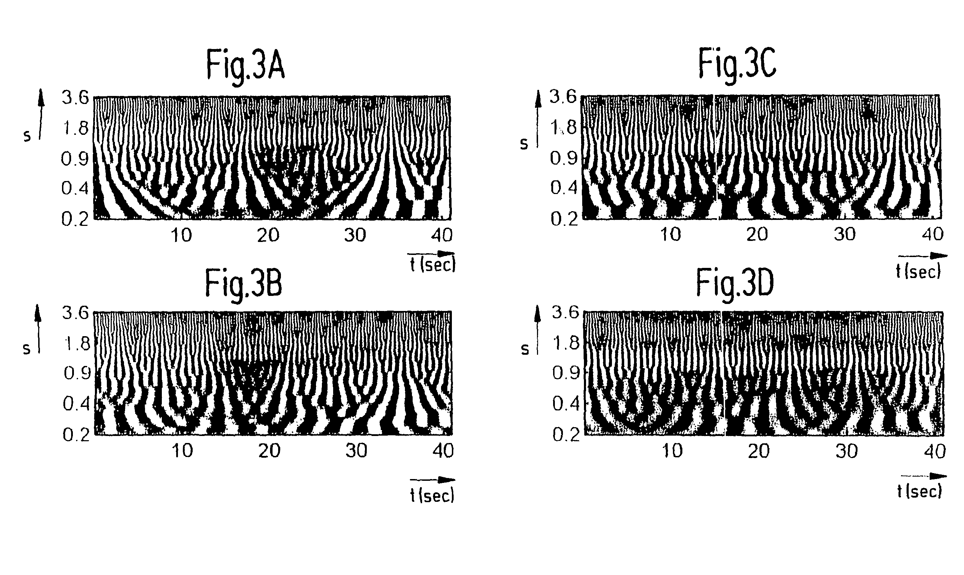

In den Fig. 3A bis 3D sind vier derartige Darstellungen zu erkennen, wobei die Abszisse die Zeitachse t und die Ordinate die Frequenz- oder Skalierungsachse s ist. Die dritte Achse der Signalintensität wird in den Darstellungen der Fig. 3A bis 3D durch unterschiedliche Schwärzung (bzw. Farbgebung) erzeugt.

Die Darstellung in Fig. 3A entspricht einer Referenzmessung für benignes Gewebe, die Darstellung in Fig. 3B einer Zentrumsmessung für benignes Gewebe, während die Fig. 3C und 3D die entsprechenden Referenz- und Zentrumsmessungen für malignes Gewebe darstellen.

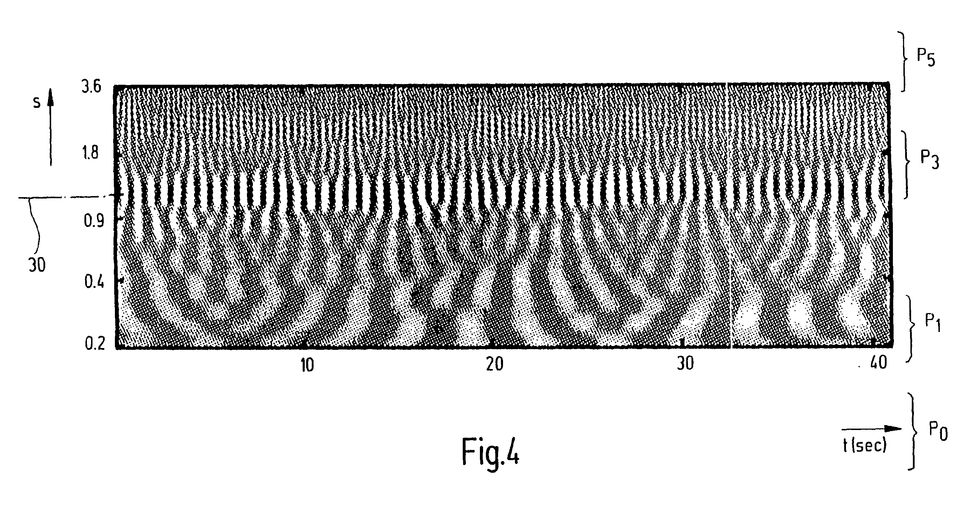

Zur weiteren Erläuterung ist die Darstellung von Fig. 3D in Fig. 4 nochmals in vergrößertem Maßstab abgebildet. In der rechten Hälfte von Fig. 4 sind vier typische Bereiche $p_0$, $p_1$, $p_3$ und $p_5$ dargestellt. Der in der eigentlichen Abbildung nicht mehr gezeigte Bereich $p_0$ entspricht der Atmung (langsame Frequenz), der über einen Übergangsbereich $p_1$ in den Bereich $p_3$ des Herzschlages (mittlere Frequenz) übergeht, an den sich weiter oben über einen weiteren Übergangsbereich der Bereich $p_5$ der reflektierten Pulswellen (hohe Frequenz) anschließt.

Auf diese Weise entsteht ein charakteristisches Muster mit periodischen Streifen, die den jeweiligen periodischen Vorgängen zugeordnet sind.

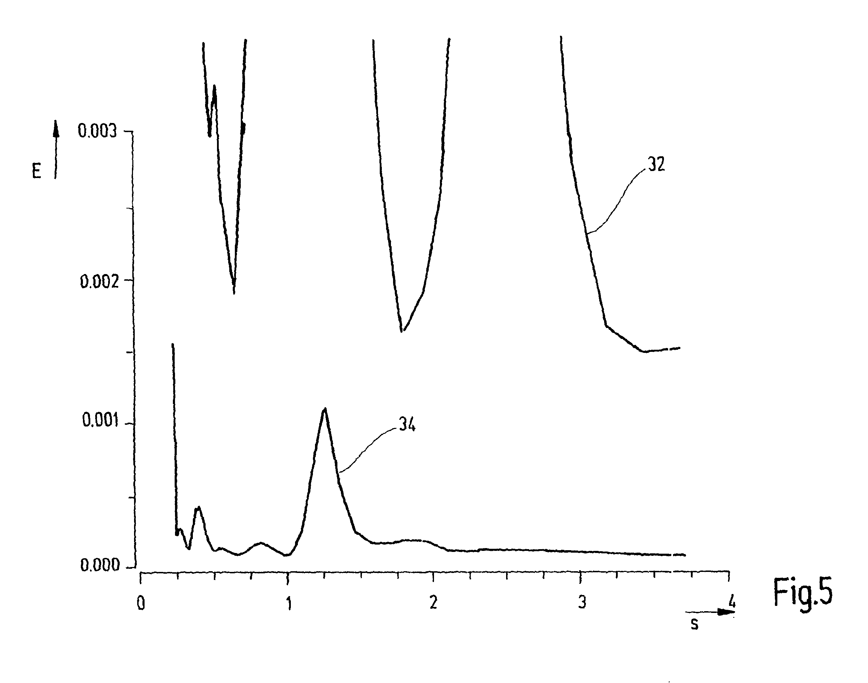

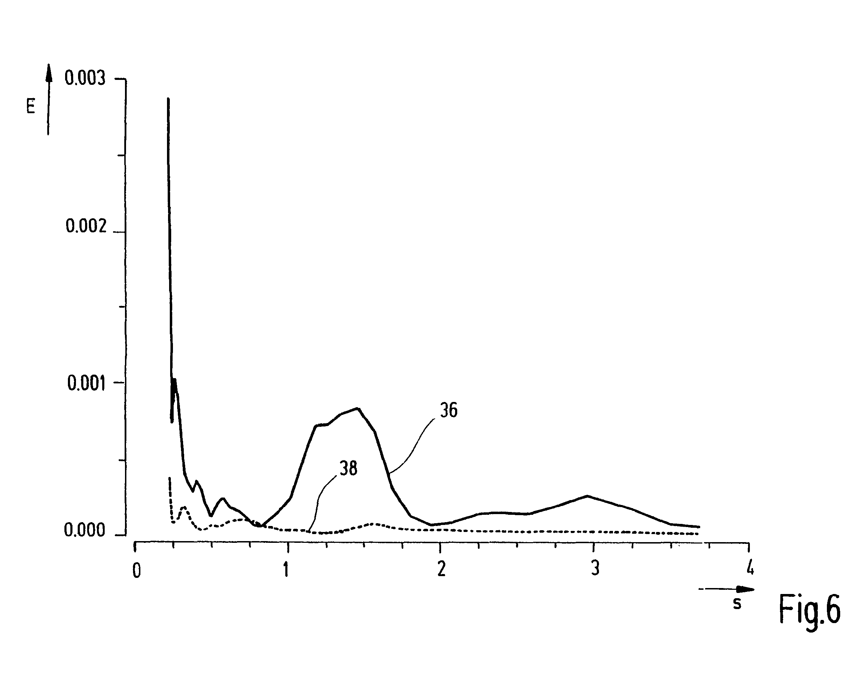

Zur Interpretation dieser Wavelet-Analyse werden auf der Frequenz- oder Skalierungsachse s sogenannte Skalierungsebenen definiert, von denen eine in Fig. 4 mit 30 im Bereich $p_3$ für den Herzschlag eingezeichnet ist. Wenn man entlang der Skalierungsebene 30 in Abszissenrichtung durch das Diagramm gemäß Fig. 4 hindurchgeht und die (vorzugsweise quadrierten) Signalwerte über der Zeitachse aufintegriert, so erhält man für jede Skalierungsebene 30 einen Wert, der im folgenden als "Vasomotionsenergie E" bezeichnet wird. Die Vasomotionsenergie E läßt sich für die verschiedenen Skalierungsebenen s auftragen, wie dies in den Fig. 5 und 6 geschehen ist.

In Fig. 5 ist mit 32 ein erster Verlauf und mit 34 ein zweiter Verlauf der Vasomotionsenergie E über der Skalierungsebene (oder Frequenzachse) s aufgetragen. Der erste Verlauf 32 gehört zu einem malignen Melanom (beispielsweise entsprechend der Wavelet-Analyse gemäß Fig. 3D bzw. gemäß Fig. 4), während der zweite Verlauf 34 dem umgebenden nicht-pigmentierten Hautbereich entspricht. Es ist deutlich erkennbar, daß sich eine signifikante Abweichung der Verläufe 32 und 34 ergibt.

Zum Vergleich zeigt Fig. 6 entsprechende Verläufe, die aus der Untersuchung von zwar ebenfalls pigmentierten, jedoch benignen Gewebebereichen im Vergleich zu umgebendem nicht-pigmentiertem Gewebe gewonnen wurden. Ein dritter Verlauf 38 entspricht dabei dem nicht-pigmentierten Umgebungsbereich, während der vierte Verlauf 36 einem melanozytischen Naevus entspricht. Mit Ausnahme des Bereichs zwischen s = 1 und s = 2 sind die Verläufe nicht so signifikant unterschiedlich wie im Falle der Fig. 5.

Um zu erläutern, wie aus derartigen Messungen ein zuverlässiger Kennwert bestimmt werden kann, sei nochmals auf die Darstellungen gemäß den Fig. 2A und 2B in Verbindung mit Fig. 1 verwiesen.

Gemäß einer ersten Variante subtrahiert man nun den Verlauf der Vasomotionsenergie E entsprechend dem zweiten Meßverlauf 22 (umgebendes nicht-pigmentiertes Gewebe) vom Verlauf der Vasomotionsenergie E des ersten Meßverlaufs 24 (Zentrum des pigmentierten Bereiches 12).

Der so gewonnene Differenzverlauf wird nun aufintegriert. Dieses Integral ist der angestrebte Kennwert, der im folgenden als ΔE bezeichnet werden soll.

Gemäß einer Variante der Erfindung subtrahiert man hingegen den Verlauf der Vasomotionsenergie E entsprechend dem zweiten Meßverlauf 22 (Randbereich) vom Verlauf der Vasomotionsenergie E des dritten Meßverlaufs 24 (umgebendes nicht-pigmentiertes Gewebe) und subtrahiert gleichermaßen den Verlauf der Vasomotionsenergie E des ersten Meßverlaufs 20 (Zentrum des pigmentierten Bereiches 12) vom Verlauf der Vasomotionsenergie E wiederum des dritten Meßverlaufs 24.

Aus den beiden so gewonnenen Differenzverläufen wird nun der Mittelwert gebildet und dieser Mittelwert aufintegriert. Dieses Integral ist der angestrebte Kennwert, der im folgenden als ΔE bezeichnet werden soll.

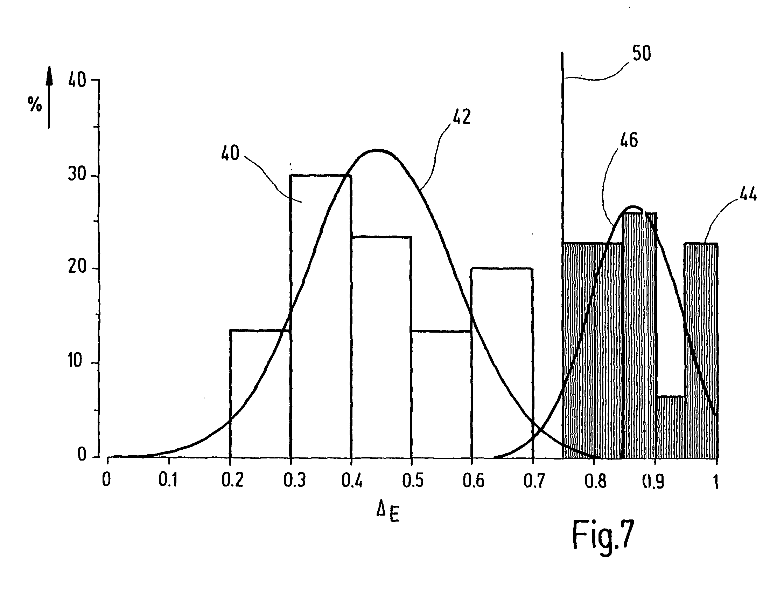

In Fig. 7 ist das Ergebnis einer klinischen Studie dargestellt, die an 61 Patienten durchgeführt wurde. Bei diesen Patienten wurden Messungen entsprechend der Darstellung in den Fig. 1 und 2 durchgeführt und diese Messungen dann in der vorstehend beschriebenen Weise ausgewertet. Bei der Bestimmung des Kennwertes ΔE wurden diese der besseren Übersichtlichkeit halber auf einen Bereich zwischen 0 und 1 normiert.

Anschließend an diese Messungen wurden den Patienten Proben an den jeweiligen pigmentierten Gewebebereichen 12 entnommen und diese histologisch untersucht.

In Fig. 7 zeigen die Balken 40 die Ergebnisse für diejenigen Patienten, bei denen im Verlauf der histologischen Untersuchung benigne Erscheinungsformen des pigmentierten Gewebebereichs gefunden wurden. So hatten z.B. etwa 30 % dieser Patienten einen Kennwert ΔE zwischen 0,3 und 0,4. Aus dem Balken 40 ließ sich eine erste Verteilungskurve 42 für diese Patienten mit benignen pigmentierten Gewebebereichen ermitteln.

Die in Fig. 7 dunkel dargestellten Balken 44 gehören demgegenüber zu denjenigen Patienten, bei denen später in der histologischen Untersuchung maligne pigmentierte Gewebebereiche festgestellt wurden. Aus dem Balken 44 ließ sich eine zweite Verteilungskurve 46 für diese Patienten ableiten.

Wie man aus Fig. 7 deutlich erkennt, sind die beiden Verteilungskurven 42 und 46 relativ scharf voneinander auf der ΔE-Achse getrennt. Es läßt sich daher ein Grenzwert 50 angeben, der bei der gewählten Normierung auf Werte zwischen 0 und 1 bei ΔE = 0,75 liegt.

Im Rahmen der vorliegenden Erfindung wird die Wavelet-Analyse bevorzugt unter Verwendung der folgenden Überlegungen ausgeführt:

Die Meßverläufe 20 und 22 gemäß Fig. 2A und 2B lassen sich analytisch wie folgt beschreiben:

$$\xi(t_n),\ t_n = \frac{n-1}{f_{samp}},\ n \in [1,N],$$

wobei $\xi(t_n)$ die Meßpunkte zu den Zeiten $t_n$ darstellt und eine konstante Abtastrate $f_{samp}$ gewählt wurde. Das Vasomotionsfeld V zum Zeitpunkt $t_\tau$ in einer Skalierungsebene s ergibt sich dann aus der Wavelet-Darstellung des Zeitverlaufs zu:

$$V(t_\tau,s) = \sum_{n \in \{1..N\}} \phi_s(t_n - t_\tau)\ \xi(t_n).$$

Die Wavelets $\phi_s$ werden aus einem sogenannten Mutter-Wavelet $\phi$ abgeleitet. Für die Analyse der Vasomotionsdaten im Rahmen der vorliegenden Erfindung erwies sich das folgende Mutter-Wavelet als besonders gut geeignet:

$$\phi(t) = \cos(17.3932 \cdot t)\, e^{-t^2} \; .$$

Dieses Mutter-Wavelet beschreibt eine örtliche Oszillation über ungefähr zehn Perioden. Aus dem Mutter-Wavelet $\phi$ werden die Wavelets wie folgt abgeleitet:

$$\phi_s(t) = \sqrt{s}\,\phi(st) .$$

$\sqrt{s}$ normalisiert die Wavelets $\phi_s$ unabhängig von der Skalierungsebene s.

Die Skalierungsebenen werden in sogenannte "Octaves" und "Voices" unterteilt:

$$s \to s_\sigma = 2^{\sigma / N^{(voices)}} \; mit \quad \sigma \in \left\{ 0..\,N^{(voices)} N^{(octaves)} \right\} .$$

$N^{(octaves)}$ ist die Anzahl der Octaves im Vasomotionsfeld und $N^{(voices)}$ ist die Anzahl der Voices pro Octave. Dies führt zur sogenannten kontinuierlichen Wavelet-Transformation.

Die Voices und Octaves der Skalierungsebenen sind eng mit den Frequenzen eines Fourier-Spektrums verknüpft. Zum besseren Verständnis werden die Skalierungswerte in Einheiten angegeben, die so eng wie möglich bei den Fourier-Frequenzen liegen. Es ergeben sich dann fünf verschiedene Skalierungsbereiche, wie bereits weiter oben zu Fig. 4 erläutert wurde:

| p0: | $S_\sigma$ = 0,15-0,31 | (Atmung); |
|---|---|---|
| p1: | $S_\sigma$ = 0,31-0,62 | (Übergang von der Atmung zum Herzschlag); |
| p2: | $S_\sigma$ = 0,62-1,20 | (Übergang von der Atmung zum Herzschlag); |
| p3: | $S_\sigma$ = 1,20-2,50 | (Herzschlag); |
| p4: | $S_\sigma$ = 2,50-5,00 | (Übergang vom Herzschlag zu reflektierten Pulswellen); |
| p5: | $S_\sigma$ = 5,00-10,00 | (reflektierte Pulswellen); |

Insgesamt ergibt sich dann das Vasomotionsfeld V als:

$$V_{s_\sigma} = \sum_{n \in \{1..N\}} \phi_{s_\sigma}(t_n - t_r)\, \xi(t_n) .$$

Derartige Vasomotionsfelder V sind in den Fig. 3A bis 3D und 4 als zweidimensionale Ausdrucke mit Grauwert- oder farbcodierten Amplituden dargestellt.

Zur weiteren Auswertung der Signale wird die Vasomotions-Feldenergie $E_{S_\sigma}$ für jede Skalierungsebene $S_\sigma$ ermittelt, indem über die Zeit $\tau$, aufsummiert wird.

$$E_{s_r} = \sum_{r \in N} V_{s_\sigma}^2 .$$

Zum Vergleich der unterschiedlichen vasomotionsfelder V, insbesondere zwischen gesundem und pigmentiertem Gewebe, wird die Energiedifferenz bzw. der Kennwert $\Delta E$ wie folgt bestimmt:

$$\Delta E = C^{-1} \sum_{s,\sigma} \left| E_{s_\sigma}^{(gesund)} - E_{s_\sigma}^{(pigmentiert)} \right|,$$

$$C = \sum_{s,\sigma} \left| E_{s_\sigma}^{(gesund)} + E_{s_\sigma}^{(pigmentiert)} \right| \quad \Rightarrow \quad \Delta E \in [0,1].$$

**Patentansprüche**

1. Verfahren zum Bestimmen eines Kennwertes ($\Delta E$) für die Durchblutung von veränderten Gewebebereichen (12) in Organen von Lebewesen mit den Schritten:

   a) Bestimmen eines ersten Volumenstroms des Blutes mittels Laser-Fluxmetrie in einem ersten Meßpunkt (14) innerhalb des veränderten Gewebebereichs (12) als erster Meßverlauf (20) eines Volumenstrom-Signals (LDF) in Abhängigkeit von der Zeit (t);
   b) Bestimmen eines zweiten Volumenstroms des Blutes mittels Laser-Fluxmetrie in einem zweiten Meßpunkt (18) innerhalb eines nicht-veränderten Gewebebereichs als zweiter Meßverlauf (22) eines Volumenstrom-Signals (LDF) in Abhängigkeit von der Zeit (t);
   c) Bestimmen eines dritten Volumenstroms des Blutes mittels Laser-Fluxmetrie in einem dritten Meßpunkt (16) im Übergang zwischen dem veränderten Gewebebereich (12) und dem umgebenden, nicht-veränderten Gewebebereich als dritter Meßverlauf eines Volumenstrom-Signals (LDF) in Abhängigkeit von der Zeit (t);
   d) Ausführen jeweils einer Wavelet-Analyse der Meßverläufe (20, 22) als dreidimensionale Darstellung der Signalintensität über einer Frequenz- oder Skalierungsachse (s) und der Zeit (t);
   e) Bestimmen jeweils des Verlaufs einer Vasomotionsenergie (E) über der Frequenz- oder Skalierungsachse (s) für die der Wavelet-Analyse unterworfenen: Meßverläufe (20, 22), wobei die Vasomotionsenergie (E) das Integral der Signalintensität über der Zeit für einen bestimmten Frequenz- oder Skalierungswert (s) ist;
   f) Subtrahieren des Verlaufs der Vasomotionsenergie (E) des dritten Meßverlaufs vom Verlauf der Vasomotionsenergie (E) des zweiten Meßverlaufs (22), derart, daß ein erster Differenzverlauf gebildet wird;
   g) Subtrahieren des Verlaufs der Vasomotionsenergie (E) des ersten Meßverlaufs vom Verlauf der Vasomotionsenergie (E) des zweiten Meßverlaufs (22), derart, daß ein zweiter Differenzverlauf gebildet wird;
   h) Bilden eines Mittelwertes des ersten und des zweiten Differenzverlaufs;
   i) Aufintegrieren des Mittelwertes zum Erhalten des Kennwertes ($\Delta E$).

2. Verfahren nach Anspruch 1, **dadurch gekennzeichnet, daß** der Kennwert ($\Delta E$) mit einem Referenzwert (50) verglichen wird.

3. Verfahren nach Anspruch 1 oder 2, **dadurch gekennzeichnet, daß** der erste Meßpunkt (14) in einen pigmentierten Gewebebereich (12) gelegt wird.

4. Verfahren nach einem oder mehreren der Ansprüche 1 bis 3, **dadurch gekennzeichnet, daß** die Gewebebereiche (12) Bereiche der Haut (10) des Lebewesens sind.

5. Vorrichtung zum Bestimmen eines Kennwertes ($\Delta E$) für die Durchblutung von veränderten Gewebebereichen (12) in Organen von Lebewesen mit:

   a) einem Laser-Fluxmeter zum Bestimmen

      - eines ersten Volumenstroms des Blutes in einem ersten Meßpunkt (14) innerhalb des veränderten Gewebebereichs (12) als erster Meßverlauf (20) eines Volumenstrom-Signals (LDF) in Abhängigkeit von der Zeit (t);
      - eines zweiten Volumenstroms des Blutes in einem zweiten Meßpunkt (18) innerhalb eines nicht-veränderten Gewebebereichs als zweiter Meßverlauf (22) eines Volumenstrom-Signals (LDF) in Abhängigkeit von der Zeit (t);
      - eines dritten Volumenstroms des Blutes in einem dritten Meßpunkt (16) im Übergang zwischen dem veränderten Gewebebereich (12) und dem umgebenden, nicht-veränderten Gewebebereich als dritter Meßverlauf eines Volumenstrom-Signals (LDF) in Abhängigkeit von der Zeit (t);

b) Mitteln zum Ausführen jeweils einer Wavelet-Analyse der Meßverläufe (20, 22) als dreidimensionale Darstellung der Signalintensität über einer Frequenz- oder Skalierungsachse (s) und der Zeit (t);
c) Mitteln zum Bestimmen jeweils des Verlaufs einer Vasomotionsenergie (E) über der Frequenz- oder Skalierungsachse (s) für die der Wavelet-Analyse unterworfenen Meßverläufe (20, 22), wobei die Vasomotionsenergie (E) das Integral der Signalintensität über der Zeit für einen bestimmten Frequenz- oder Skalierungswert (s) ist;
d) Mitteln zum Subtrahieren des Verlaufs der Vasomotionsenergie (E) des dritten Meßverlaufs vom Verlauf der Vasomotionsenergie (E) des zweiten Meßverlaufs (22), derart, daß ein erster Differenzverlauf gebildet wird;
e) Mitteln zum Subtrahieren des Verlaufs der Vasomotionsenergie (E) des ersten Meßverlaufs (20) vom Verlauf der Vasomotionsenergie (E) des zweiten Meßverlaufs (22), derart, daß ein zweiter Differenzverlauf gebildet wird;
f) Mitteln zum Bilden eines Mittelwertes des ersten und des zweiten Differenzverlaufs;
g) Mitteln zum Aufintegrieren des Mittelwertes zum Erhalten des Kennwertes ($\Delta$E).

6. Vorrichtung nach Anspruch 5, **gekennzeichnet durch** Mittel zum Vergleichen des Kennwertes ($\Delta$E) mit einem Referenzwert (50).

7. Vorrichtung nach Anspruch 5 oder 6, **dadurch gekennzeichnet, daß** der erste Meßpunkt (14) in den pigmentierten Gewebebereich (12) gelegt wird.

8. Vorrichtung nach einem oder mehreren der Ansprüche 5 bis 7, **dadurch gekennzeichnet, daß** die Gewebebereiche (12) Bereiche der Haut (10) des Lebewesens sind.

**Claims**

1. A method for determining a characteristic value ($\Delta$E) for the perfusion of modified tissue regions (12) in organs of living beings, having the following steps:

a) determining a first volumetric flow rate of the blood by means of laser flowmetry at a first measurement point (14) within the modified tissue region (12) as a first measurement profile (20) of a volumetric flow rate signal (LDF) as a function of time (t);
b) determining a second volumetric flow rate of the blood by means of laser flowmetry at a second measurement point (18) within an unmodified tissue region as a second measurement profile (22) of a volumetric flow rate signal (LDF) as a function of time (t);
c) determining a third volumetric flow rate of the blood by means of laser flowmetry at a third measurement point (16) at the transition between the modified tissue region (12) and the surrounding, unmodified tissue region as a third measurement profile of a volumetric flow rate signal (LDF) as a function of time (t);
d) carrying out a wavelet analysis of each of the measurement profiles (20, 22) as a three-dimensional representation of the signal intensity over a frequency or scaling axis (s) and time (t);
e) determining the profile of a vasomotion energy (E) over the frequency or scaling axis (s) for each of the measurement profiles (20, 22) subjected to the wavelet analysis, the vasomotion energy (E) being the integral of the signal intensity with respect to time for a particular frequency or scaling value (s);
f) subtracting the profile of the vasomotion energy (E) of the third measurement profile from the profile of the vasomotion energy (E) of the second measurement profile (22), so as to form a first difference profile;
g) subtracting the profile of the vasomotion energy (E) of the first measurement profile from the profile of the vasomotion energy (E) of the second measurement profile (22), so as to form a second difference profile;
h) forming an average value of the first and second difference profiles;
i) integrating the average value in order to obtain the characteristic value $\Delta$E).

2. The method of claim 1, **characterized in that** the characteristic value ($\Delta$E) is compared with a reference value (50).

3. The method of claim 1 or 2, **characterized in that** the first measurement point (14) is located in a pigmented tissue region (12).

4. The method of one or several of claims 1 to 3, **characterized in that** the tissue regions (12) are regions of the skin (10) of the living being.

5. A device for determining a characteristic value ($\Delta$E) for the perfusion of modified tissue regions (12) in organs of living beings, having:

a) a laser flowmeter for determining

- a first volumetric flow rate of the blood at a first measurement point (14') within the modified tissue region (12') as a first measurement profile (20) of a volumetric flow rate signal (LDF) as a function of time (t);
- a second volumetric flow rate of the blood at a second measurement point (18) within an unmodified tissue region as a second measurement profile (22) of a volumetric flow rate signal (LDF) as a function of time (t);
- a third volumetric flow rate of the blood at a third measurement point (16) at the transition between the modified tissue region (12) and the surrounding, unmodified tissue region as a third measurement profile of a volumetric flow rate signal (LDF) as a function of time (t);

b) means for carrying out a wavelet analysis of each of the measurement profiles (20, 22) as a three-dimensional representation of the signal intensity over a frequency or scaling axis (s) and time (t);

c) means for determining the profile of a vasomotion energy (E') over the frequency or scaling axis (s) for each of the measurement profiles (20, 22) subjected to the wavelet analysis, the vasomotion energy (E) being the integral of the signal intensity with respect to time for a particular frequency or scaling value (s);

d) means for subtracting the profile of the vasomotion energy (E) of the third measurement profile from the profile of the vasomotion energy (E) of the second measurement profile (22), so as to form a first difference profile;

e) means for subtracting the profile of the vasomotion energy (E) of the first measurement profile (20) from the profile of the vasomotion energy (E) of the second measurement profile (22), so as to form a second difference profile;

f) means for forming an average value of the first and second difference profiles;

g) means for integrating the average value in order to obtain the characteristic value ($\Delta$E).

6. The device of claim 5, **characterized in that** means for comparing the characteristic value ($\Delta$E) with a reference value (50).

7. The device of claim 5 or 6, **characterized in that** the first measurement point (14) is located in the pigmented tissue region (12).

8. The device one or several of claims 5 to 7, **characterized in that** the tissue regions (12) are regions of the skin (10) of the living being.

## Revendications

1. Procédé permettant de déterminer une valeur caractéristique ($\Delta$E) pour la circulation sanguine de régions tissulaires modifiées (12) dans des organes d'êtres vivants, comportant les étapes suivantes :

a) détermination d'un premier courant volumique du sang au moyen de la fluxmétrie par laser à un premier point de mesure (14) à l'intérieur de la région tissulaire modifiée (12) comme premier tracé (20) d'un signal de courant volumique (LDF) en fonction du temps (t) ;

b) détermination d'un deuxième courant volumique du sang au moyen de la fluxmétrie par laser à un deuxième point de mesure (18) à l'intérieur d'une région tissulaire non modifiée comme deuxième tracé (22) d'un signal de courant volumique (LDF) en fonction du temps (t) ;

c) détermination d'un troisième courant volumique du sang au moyen de la fluxmétrie par laser à un troisième point de mesure (16) situé à la transition entre la région tissulaire modifiée (12) et la région tissulaire non modifiée environnante comme troisième tracé d'un signal de courant volumique (LDF) en fonction du temps (t) ;

d) réalisation d'une analyse en ondelette de chaque tracé de mesure (20, 22) sous forme de représentation tridimensionnelle de l'intensité des signaux sur un axe de fréquence ou d'échelle (s) et dans le temps (t) ;

e) détermination du tracé respectif d'une énergie de vasomotion (E) sur l'axe de fréquence ou d'échelle (s) pour les tracés de mesures (20, 22) soumis a l'analyse en ondelette, l'énergie de vasomotion (E) étant l'intégrale de l'intensité des signaux dans le temps pour une valeur de fréquence ou d'échelle (s) déterminée ;

f) soustraction du tracé de l'énergie de vasomotion (E) du troisième tracé de mesure du tracé de l'énergie de vasomotion (E) du deuxième tracé de mesure (22) de manière à former un premier tracé différentiel ;

g) soustraction du tracé de l'énergie de vasomotion (E) du premier tracé de mesure du tracé de l'énergie de vasomotion (E) du deuxième tracé de mesure (22) de manière à former un deuxième tracé différentiel ;

h) formation d'une valeur moyenne du premier et du deuxième tracé différentiel ;

i) intégration de la valeur moyenne pour obtenir la valeur caractéristique ($\Delta$E).

**2.** Procédé selon la revendication 1, **caractérisé en ce que** la valeur caractéristique (ΔE) est comparée à une valeur de référence (50).

**3.** Procédé selon la revendication 1 ou 2, **caractérisé en ce que** le premier point de mesure (14) est placé dans une région tissulaire pigmentée (12).

**4.** Procédé selon une ou plusieurs des revendications 1 à 3, **caractérisé en ce que** les régions tissulaires (12) sont des régions de la peau (10) de l'être vivant.

**5.** Dispositif permettant de déterminer une valeur caractéristique (ΔE) relative à la circulation sanguine de régions tissulaires modifiées (12) dans des organes d'êtres vivants avec :

a) un fluxmètre laser permettant de déterminer

- un premier courant volumique du sang à un premier point de mesure (14) à l'intérieur de la région tissulaire modifiée (12) comme premier tracé (20) d'un signal de courant volumique (LDF) en fonction du temps (t) ;
- un deuxième courant volumique du sang à un deuxième point de mesure (18) à l'intérieur d'une région tissulaire non modifiée comme deuxième tracé (22) d'un signal de courant volumique (LDF) en fonction du temps (t) ;
- un troisième courant volumique du sang à un troisième point de mesure (16) situé à la transition entre la région tissulaire modifiée (12) et la région tissulaire non modifiée environnante comme troisième tracé d'un signal de courant volumique (LDF) en fonction du temps (t) ;

b) des moyens pour réaliser une analyse en ondelette de chaque tracé de mesure (20, 22) sous forme de représentation tridimensionnelle de l'intensité des signaux sur un axe de fréquence ou d'échelle (s) et dans le temps (t) ;

c) des moyens pour déterminer le tracé respectif d'une énergie de vasomotion (E) sur l'axe de fréquence ou d'échelle (s) pour les tracés de mesures (20, 22) soumis à l'analyse en ondelette, l'énergie de vasomotion (E) étant l'intégrale de l'intensité des signaux dans le temps pour une valeur de fréquence ou d'échelle (s) déterminée ;

d) des moyens pour soustraire le tracé de l'énergie de vasomotion (E) du troisième tracé de mesure du tracé de l'énergie de vasomotion (E) du deuxième tracé de mesure (22) de manière à former un premier tracé différentiel ;

e) des moyens pour soustraire le tracé de l'énergie de vasomotion (E) du premier tracé de mesure (20) du tracé de l'énergie de vasomotion (E) du deuxième tracé de mesure (22) de manière à former un deuxième tracé différentiel ;

f) des moyens pour former une valeur moyenne du premier et du deuxième tracé différentiel ;

g) des moyens pour intégrer la valeur moyenne afin d'obtenir la valeur caractéristique (ΔE).

**6.** Dispositif selon la revendication 5, **caractérisé par** des moyens permettant de comparer la valeur caractéristique (ΔE) et une valeur de référence (50).

**7.** Dispositif selon la revendication 5 ou 6, **caractérisé en ce que** le premier point de mesure (14) est placé dans une région tissulaire pigmentée (12).

**8.** Dispositif selon l'une quelconque ou plusieurs des revendications 5 à 7, **caractérisé en ce que** les régions tissulaires (12) sont des régions de la peau (10) de l'être vivant.

Fig.1

Fig.2A

Fig.2B

Fig.3C

Fig.3D

Fig.3A

Fig.3B

Fig.4

EP 1 416 846 B1

Fig.5

Fig.6

EP 1 416 846 B1

Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KVERNMO H.D et al.** *Microvascular Research,* 1999, vol. 57, 298-309

- **TUR E et al.** CUTANEOUS BLOOD FLOW MEASUREMENTS FOR THE DETECTION OF MALIGNANCY IN PIGMENTED SKIN LESIONS. *DERMATOLOGY,* 1992, vol. 184 (1), 8-11